(19) Europäisches Patentamt
European Patent Office
Office européen des brevets

(11) **EP 1 335 793 B1**

(12) **EUROPÄISCHE PATENTSCHRIFT**

(45) Veröffentlichungstag und Bekanntmachung des Hinweises auf die Patenterteilung:
**31.12.2014 Patentblatt 2015/01**

(51) Int Cl.:
*B01J 23/28* *(2006.01)*        *B01J 27/057* *(2006.01)*
*C07C 57/05* *(2006.01)*        *B01J 37/02* *(2006.01)*
*B01J 35/02* *(2006.01)*

(21) Anmeldenummer: **01987695.2**

(22) Anmeldetag: **16.10.2001**

(86) Internationale Anmeldenummer:
**PCT/EP2001/011909**

(87) Internationale Veröffentlichungsnummer:
**WO 2002/032571 (25.04.2002 Gazette 2002/17)**

(54) **KATALYSATOR BESTEHEND AUS EINEM TRÄGERKÖRPER UND EINER AUF DER OBERFLÄCHE DES TRÄGERKÖRPERS AUFGEBRACHTEN KATALYTISCH AKTIVEN OXIDMASSE**

CATALYST COMPRISING A SUPPORT AND A CATALYTICALLY ACTIVE OXIDE MATERIAL APPLIED TO THE SURFACE OF THE SUBSTRATE

CATALYSEUR CONSTITUE D'UN SUPPORT ET D'UNE MASSE D'OXYDE CATALYTIQUEMENT ACTIVE APPLIQUEE SUR LA SURFACE DU SUPPORT

(84) Benannte Vertragsstaaten:
**AT BE CH CY DE DK ES FI FR GB GR IE IT LI LU MC NL PT SE TR**

(30) Priorität: **17.10.2000 DE 10051419**

(43) Veröffentlichungstag der Anmeldung:
**20.08.2003 Patentblatt 2003/34**

(73) Patentinhaber: **BASF SE**
**67056 Ludwigshafen (DE)**

(72) Erfinder:
• **BORGMEIER, Frieder**
  **68163 Mannheim (DE)**
• **TENTEN, Andreas**
  **3090 Overijse (BE)**
• **HIBST, Hartmut**
  **69198 Schriesheim (DE)**

(56) Entgegenhaltungen:
EP-A- 0 608 838      EP-A2- 0 068 192
US-A- 4 524 236      US-A- 4 565 658
US-A- 6 043 185      US-A- 6 143 690

Bemerkungen:
Die Akte enthält technische Angaben, die nach dem Eingang der Anmeldung eingereicht wurden und die nicht in dieser Patentschrift enthalten sind.

**Beschreibung**

Schalenkatalysator bestehend aus einem Trägerkörper und einer auf der Oberfläche des Trägerkörpers aufgebrachten katalytisch aktiven Oxidmasse

Vorliegende Erfindung betrifft einen Schalenkatalysator, der aus einem Trägerkörper und einer auf der Oberfläche des Trägerkörpers aufgebrachten katalytisch aktiven Oxidmasse der allgemeinen Formel I,

$$Mo_1V_bM^1_cM^2_dO_n \qquad (I),$$

mit

$M^1$ = Te,

$M^2$ = wenigstens eines der Elemente aus der Gruppe umfassend Nb, Ta, W, Ti, Al, Zr, Cr, Mn, Ga, Fe, Ru, Co, Rh, Ni, Pd, Pt, Bi, B und Ce,

b = 0,01 bis 1,

c = 0,01 bis 1,

d = 0,01 bis 1 und

n = eine Zahl, die unter der Voraussetzung der Ladungsneutralität durch die Wertigkeit und Häufigkeit der von Sauerstoff verschiedenen Elemente in (I) bestimmt wird,

besteht.

Außerdem betrifft vorliegende Erfindung die Verwendung der vorstehend beschriebenen Schalenkatalysatoren als Katalysatoren für die heterogen katalysierte Gasphasenoxidation von Propan zu Acrylsäure.

Mulitmetalloxidmassen, die eine der allgemeinen Formel I entsprechende Stöchiometrie aufweisen, sind bekannt (vgl. z. B. EP-A 608838, EP-A 529853, JP-A 7-232071, JP-A 10-57813, JP-A 2000-37623, JP-A 10-36311, Procedings ISO'99, Rimini (Italy), Sept. 10-11, 1999, G. Centi and S. Perathoner Ed., SCI Pub. 1999, EP-A 767164, Catalysis Today 49(1999), S. 141 - 153, EP-A 962253, Applied Catalysis A: General 194 bis 195 (2000), S. 479 bis 485, JP-A 11/169716, EP-A 895809 und DE-A 19835247) und wurden auch schon in den älteren Anmeldungen DE-A 10029338 und DE-A 10046672 vorgeschlagen. Multimetalloxidmassen, die eine chemische Zusammensetzung wie die Oxidmassen der allgemeinen Formel (I) aufweisen, sind auch aus der WO 00/29106 bekannt.

Im vorstehend zitierten Stand der Technik wird auch bereits vorgeschlagen, Multimetalloxidmassen einer solchen Zusammensetzung als Katalysatoren für die heterogen katalysierte Gasphasenoxidation von Propan zu Acrylsäure zu verwenden.

Acrylsäure ist eine wichtige ethylenisch ungesättigte Verbindung, die sowohl als solche als auch in Gestalt ihrer Alkylester zur Herstellung von Polymerisaten Verwendung findet.

Allen Schriften des zitierten Standes der Technik ist gemein, dass sie die Multimetalloxidmassen der allgemeinen Formel (I) in versplitteter Form zur Katalyse der Gasphasenoxidation des Propans zu Acrylsäure einsetzen.

Dadurch weist die Katalysatorbeschickung zwar eine erhöhte Aktivität auf, was im Fall des vergleichsweise reaktionsträgen Propans zweckmäßig ist, doch vermag die wesentlich wertvollere Katalysatoreigenschaft, nämlich die Selektivität der Acrylsäurebildung, im Fall einer Verwendung von Mulitmetalloxidmassensplitt nicht im vollen Umfang zu befriedigen.

Die DE-A 4442346 betrifft ein Verfahren zur Herstellung von Schalenkatalysatoren von Multimetalloxidmassen die denen der allgemeinen Formel (I) ähnlich sind.

Die DE-A 4442346 empfiehlt diese Schalenkatalysatoren als Katalysatoren für die gasphasenkatalytische Oxidation von Acrolein zu Acrylsäure.

Relevanten Stand der Technik bildet auch die EP-A 1090684.

Die Aufgabe der vorliegenden Erfindung bestand daher darin, die Multimetalloxidmassen (I) in einer von Split verschiedenen Form zur Verfügung zu stellen, die bei einer Verwendung als Katalysatoren für die gasphasenkatalytische Oxidation von Propan zu Acrylsäure eine erhöhte Selektivität der Acrylsäurebildung bedingt.

Als Lösung der Aufgabe wurde ein Schalenkatalysator gefunden, der aus einem Trägerkörper und einer auf der Oberfläche des Trägerkörpers aufgebrachten katalytisch aktiven Oxidmasse der allgemeinen Formel I,

$$Mo_1V_bM^1_cM^2_dO_n \qquad (I),$$

mit

M$^1$ = Te,

M$^2$ = wenigstens eines der Elemente aus der Gruppe umfassend Nb, Ta, W, Ti, Al, Zr, Cr, Mn, Ga, Fe, Ru, Co, Rh, Ni, Pd, Pt, Bi, B und Ce,

b = 0,01 bis 1,

c = 0,01 bis 1,

d = 0,01 bis 1 und

n = eine Zahl, die unter der Voraussetzung der Ladungsneutralität durch die Wertigkeit und Häufigkeit der von Sauerstoff verschiedenen Elemente in (I) bestimmt wird,

besteht und dadurch gekennzeichnet ist, dass die Längstausdehnung des Trägerkörpers 1 bis 10 mm und die Dicke der aktiven Oxidmassenschale 10 bis 700 mm beträgt.

Erfindungsgemäß ist die Verwendung von Oxidmassen der allgemeinen Formel (I) mit M$^1$ = Te. Ferner ist es erfindungsgemäß günstig, wenn M$^2$ = Nb, Ta, W und/oder Titan ist. Vorzugsweise ist M$^2$ = Nb.

Der stöchiometrische Koeffizient b der erfindungsgemäß zu verwendenden Oxidmassen der allgemeinen Formel (I) beträgt mit Vorteil 0,1 bis 0,6. In entsprechender weise beläuft sich der Vorzugsbereich für den stöchiometrischen Koeffizienten c auf 0, 05 bis 0,4 und günstige Werte für d betragen 0,1 bis 0, 6. Besonders günstige erfindungsgemäß zu verwendende Oxidmassen der allgemeinen Formel (I) sind solche, bei denen die stöchiometrischen Koeffizienten b, c und d simultan in den vorgenannten Vorzugsbereichen liegen.

Die erfindungsgemäß zu verwendenden Trägerkörper sind vorzugsweise chemisch inert, d.h., sie greifen in den Ablauf der katalytischen Gasphasenoxidation des Propans zu Acrylsäure, die durch die erfindungsgemäßen Schalenkatalysatoren katalysiert wird, im wesentlichen nicht ein. Als Material für die Trägerkörper kommen erfindungsgemäß insbesondere Aluminiumoxid, Siliciumdioxid, Silicate wie Ton, Kaolin, Steatit, Bims, Aluminiumsilicat und Magnesiumsilicat, Siliciumcarbid, Zirkondioxid und Thoriumdioxid in Betracht.

Die Oberfläche des Trägerkörpers kann sowohl glatt als auch rauh sein. Mit Vorteil ist die Oberfläche des Trägerkörpers rauh, da eine erhöhte Oberflächenrauhigkeit in der Regel eine erhöhte Haftfestigkeit der aufgebrachten Aktivmassenschale bedingt.

Häufig liegt die Oberflächenrauhigkeit $R_z$ des Trägerkörpers im Bereich von 5 bis 200 $\mu$m, oft im Bereich von 20 bis 100 $\mu$m (bestimmt gemäß DIN 4768 Blatt 1 mit einem "Hommel Tester für DIN-ISO Oberflächenmeßgrößen" der Fa. Hommelwerke, DE).

Ferner kann das Trägermaterial porös oder unporös sein. Zweckmäßigerweise ist das Trägermaterial unporös (Gesamtvolumen der Poren auf das Volumen des Trägerkörpers bezogen ≤ 1 Vol.-%).

Die Dicke der auf den erfindungsgemäßen Schalenkatalysatoren befindlichen aktiven Oxidmassenschale liegt bei 10 bis 700 $\mu$m. Sie kann aber auch 100 bis 700 $\mu$m, 200 bis 600 $\mu$m oder 300 bis 500 $\mu$m betragen. Mögliche Schalendicken sind auch 10 bis 500 $\mu$m, 100 bis 500 $\mu$m oder 200 bis 300 $\mu$m.

Prinzipiell kommen für das erfindungsgemäße Verfahren beliebige Geometrien der Trägerkörper in Betracht. Ihre Längstausdehnung beträgt 1 bis 10 mm. Vorzugsweise werden jedoch Kugeln oder Zylinder, insbesondere Hohlzylinder, als Trägerkörper angewendet. Günstige Durchmesser für Trägerkugeln betragen 1,5 bis 4 mm. Werden Zylinder als Trägerkörper verwendet, so beträgt deren Länge vorzugsweise 2 bis 10 mm und ihr Außendurchmesser bevorzugt 4 bis 10 mm. Im Fall von Ringen liegt die Wanddicke darüber hinaus üblicherweise bei 1 bis 4 mm. Erfindungsgemäß geeignete ringförmige Trägerkörper können auch eine Länge von 3 bis 6 mm, einen Außendurchmesser von 4 bis 8 mm und eine Wanddicke von 1 bis 2 mm aufweisen. Möglich ist aber auch eine Trägerringgeometrie von 7 mm x 3 mm x 4 mm oder von 5 mm x 3 mm x 2 mm (Außendurchmesser x Länge x Innendurchmesser).

Die Herstellung erfindungsgemäßer Schalenkatalysatoren kann in einfachster Weise so erfolgen, dass man aktive Oxidmassen der allgemeinen Formel (I) vorbildet, sie in eine feinteilige Form überführt und abschließend mit Hilfe eines flüssigen Bindemittels auf die Oberfläche des Trägerkörpers aufbringt. Dazu wird die Oberfläche des Trägerkörpers in einfachster Weise mit dem flüssigen Bindemittel befeuchtet und durch Inkontaktbringen mit feinteiliger aktiver Oxidmasse der allgemeinen Formel (I) eine Schicht der Aktivmasse auf der befeuchteten Oberfläche angeheftet. Abschließend wird der beschichtete Trägerkörper getrocknet. Selbstredend kann man zur Erzielung einer erhöhten Schichtdicke den Vorgang periodisch wiederholen. In diesem Fall wird der beschichtete Grundkörper zum neuen "Trägerkörper" etc..

Die Feinheit der auf die Oberfläche des Trägerkörpers aufzubringenden katalytisch aktiven Oxidmasse der allgemeinen Formel (I) wird selbstredend an die gewünschte Schalendicke angepaßt. Für den Schalendickenbereich von 100 bis 500 $\mu$m eignen sich z. B. solche Aktivmassenpulver, von denen wenigstens 50 % der Gesamtzahl der Pulverpartikel ein Sieb der Maschenweite 1 bis 20 $\mu$m passieren und deren numerischer Anteil an Partikeln mit einer Längstausdehnung oberhalb von 50 $\mu$m weniger als 10 % beträgt. In der Regel entspricht die Verteilung der Längstausdehnungen der Pulverpartikel herstellungsbedingt einer Gaußverteilung.

Für eine Durchführung des beschriebenen Beschichtungsverfahrens im technischen Maßstab empfiehlt sich z. B. die Anwendung des in der DE-A 2909671 offenbarten Verfahrensprinzips. D.h., die zu beschichtenden Trägerkörper werden in einem vorzugsweise geneigten (der Neigungswinkel beträgt in der Regel $\geq$ 0° und $\leq$ 90°, meist $\geq$ 30° und $\leq$ 90°; der Neigungswinkel ist der Winkel der Drehbehältermittelachse gegen die Horizontale) rotierenden Drehbehälter (z. B. Drehteller oder Dragiertrommel) vorgelegt. Der rotierende Drehbehälter führt die z. B. kugelförmigen oder zylindrischen Trägerkörper unter zwei in bestimmtem Abstand aufeinanderfolgend angeordneten Dosiervorrichtungen hindurch. Die erste der beiden Dosiervorrichtungen entspricht zweckmäßig einer Düse (z.B. eine mit Druckluft betriebene Zerstäuberdüse), durch die die im rotierenden Drehteller rollenden Trägerkörper mit dem flüssigen Bindemittel besprüht und kontrolliert befeuchtet werden. Die zweite Dosiervorrichtung befindet sich außerhalb des Zerstäubungskegels des eingesprühten flüssigen Bindemittels und dient dazu, die feinteilige oxidische Aktivmasse zuzuführen (z.B. über eine Schüttelrinne oder eine Pulverschnecke). Die kontrolliert befeuchteten Trägerkugeln nehmen das zugeführte Aktivmassenpulver auf, das sich durch die rollende Bewegung auf der äußeren Oberfläche des z. B. zylinder- oder kugelförmigen Trägerkörper zu einer zusammenhängenden Schale verdichtet.

Bei Bedarf durchläuft der so grundbeschichtete Trägerkörper im Verlauf der darauffolgenden Umdrehung wiederum die Sprühdüsen, wird dabei kontrolliert befeuchtet, um im Verlauf der Weiterbewegung eine weitere Schicht feinteiliger oxidischer Aktivmasse aufnehmen zu können usw. (eine Zwischentrocknung ist in der Regel nicht erforderlich). Feinteilige oxidische Aktivmasse und flüssiges Bindemittel werden dabei in der Regel kontinuierlich und simultan zugeführt.

Die Entfernung des flüssigen Bindemittels kann nach beendeter Beschichtung z. B. durch Einwirkung von heißen Gasen, wie $N_2$ oder Luft, erfolgen. Bemerkenswerterweise bewirkt das beschriebene Beschichtungsverfahren sowohl eine voll befriedigende Haftung der aufeinanderfolgenden Schichten aneinander, als auch der Grundschicht auf der Oberfläche des Trägerkörpers.

Wesentlich für die vorstehend beschriebene Beschichtungsweise ist, dass die Befeuchtung der zu beschichtenden Oberfläche des Trägerkörpers in kontrollierter Weise vorgenommen wird. Kurz ausgedrückt heißt dies, dass man die Trägeroberläche zweckmäßig so befeuchtet, dass diese zwar flüssiges Bindemittel adsorbiert aufweist, aber auf der Trägeroberfläche keine Flüssigphase als solche visuell in Erscheinung tritt. Ist die Trägerkörperoberfläche zu feucht, agglomeriert die feinteilige katalytisch aktive Oxidmasse zu getrennten Agglomeraten, anstatt auf die Oberfläche aufzuziehen. Detaillierte Angaben hierzu finden sich in der DE-A 2909671.

Die vorerwähnte abschließende Entfernung des verwendeten flüssigen Bindemittels kann in kontrollierter Weise z. B. durch Verdampfen und/oder Sublimieren vorgenommen werden. Im einfachsten Fall kann dies durch Einwirkung heißer Gase entsprechender Temperatur (häufig 50 bis 300, häufig 150°C) erfolgen. Durch Einwirkung heißer Gase kann aber auch nur eine Vortrocknung bewirkt werden. Die Endtrocknung kann dann beispielsweise in einem Trockenofen beliebiger Art (z. B. Bandtrockner) oder im Reaktor erfolgen. Die einwirkende Temperatur sollte dabei nicht oberhalb der zur Herstellung der oxidischen Aktivmasse angewendeten Calcinationstemperatur liegen. Selbstverständlich kann die Trocknung auch ausschließlich in einem Trockenofen durchgeführt werden.

Als Bindemittel für den Beschichtungsprozeß können unabhängig von der Art und der Geometrie des Trägerkörpers verwendet werden: Wasser, einwertige Alkohole wie Ethanol, Methanol, Propanol und Butanol, mehrwertige Alkohole wie Ethylenglykol, 1,4-Butandiol, 1,6-Hexandiol oder Glycerin, ein- oder mehrwertige organische Carbonsäuren wie Propionsäure, Oxalsäure, Malonsäure, Glutarsäure oder Maleinsäure, Aminoalkohole wie Ethanolamin oder Diethanolamin sowie ein- oder mehrwertige organische Amide wie Formamid. Günstige Bindemittel sind auch Lösungen, bestehend aus 20 bis 90 Gew.-% Wasser und 10 bis 80 Gew.-% einer in Wasser gelösten organischen Verbindung, deren Siedepunkt oder Sublimationstemperatur bei Normaldruck (1 atm) > 100°C, vorzugsweise > 150°C, beträgt. Mit Vorteil wird die organische Verbindung aus der vorstehenden Auflistung möglicher organischer Bindemittel ausgewählt. Vorzugsweise beträgt der organische Anteil an vorgenannten wäßrigen Bindemittellösungen 10 bis 50 und besonders bevorzugt 20 bis 30 Gew.-%. Als organische Komponenten kommen dabei auch Monosaccharide und Oligosaccharide wie Glucose, Fructose, Saccharose oder Lactose sowie Polyethylenoxide und Polyacrylate in Betracht.

Die Herstellung von katalytisch aktiven Oxidmassen der allgemeinen Formel (I) kann in an sich bekannter Weise wie in den eingangs zitierten Schriften des Standes der Technik erfolgen. D.h., die Herstellung kann z. B. sowohl hydrothermal, wie es z.B. die DE-A 10033121 beschreibt, als auch auf konventionelle Art und Weise erfolgen.

Im letzteren Fall sind die katalytisch aktiven Oxidmassen der allgemeinen Formel (I) dadurch erhältlich, dass man von geeigneten Quellen ihrer elementaren Konstituenten ein möglichst inniges, vorzugsweise feinteiliges Trockengemisch erzeugt und dieses bei Temperaturen von 350 bis 700°C bzw. 400 bis 650°C oder 400 bis 600°C thermisch behandelt. Die thermische Behandlung kann sowohl unter oxidierender, reduzierender als auch unter inerter Atmosphäre erfolgen. Als oxidierende Atmosphäre kommt z.B. Luft, mit molekularem Sauerstoff angereicherte Luft oder an molekularem Sauerstoff entreicherte Luft in Betracht. Vorzugsweise wird die thermische Behandlung unter inerter Atmosphäre, d.h., z.B. unter molekularem Stickstoff und/oder Edelgas, durchgeführt. Üblicherweise erfolgt die thermische Behandlung bei Normaldruck (1 atm). Selbstverständlich kann die thermische Behandlung auch unter Vakuum oder unter leichtem überatmosphärischem Druck erfolgen.

Erfolgt die thermische Behandlung unter gasförmiger Atmosphäre, kann diese sowohl stehen als auch fließen. Insgesamt kann die thermische Behandlung bis zu 24 h oder mehr in Anspruch nehmen.

Bevorzugt erfolgt die thermische Behandlung zunächst unter oxidierender (Sauerstoff enthaltender) Atmosphäre (z.B. unter Luft) bei einer Temperatur von 150 bis 400°C bzw. 250 bis 350°C. Im Anschluß daran wird die thermische Behandlung zweckmäßig unter Inertgas (z. B. molekularem Stickstoff) bei Temperaturen von 350 bis 700°C bzw. 400 bis 650°C oder 400 bis 600°C fortgesetzt. Selbstredend kann die thermische Behandlung auch so erfolgen, daß die Katalysatorvorläufermasse vor ihrer thermischen Behandlung zunächst (gegebenenfalls nach Pulverisierung) tablettiert (gegebenenfalls unter Zusatz von 0,5 bis 2 Gew.-% an feinteiligem Graphit), dann thermisch behandelt und nachfolgend wieder zerkleinert wird.

Das innige Vermischen der Ausgangsverbindungen im Rahmen der Herstellung von katalytisch aktiven Oxidmassen der allgemeinen Formel (I) kann in trockener oder in nasser Form erfolgen.

Erfolgt es in trockener Form, werden die Ausgangsverbindungen zweckmäßigerweise als feinteilige Pulver eingesetzt und nach dem Mischen und gegebenenfalls Verdichten der Calcinierung (thermischen Behandlung) unterworfen.

Vorzugsweise erfolgt das innige Vermischen jedoch in nasser Form. Üblicherweise werden die Ausgangsverbindungen dabei in Form einer wäßrigen Lösung und/oder Suspension miteinander vermischt. Anschließend wird diese wäßrige Masse getrocknet und nach der Trocknung calciniert. Zweckmäßigerweise handelt es sich bei der wäßrigen Masse um eine wäßrige Lösung. Vorzugsweise erfolgt der Trocknungsprozeß unmittelbar im Anschluß an die Herstellung der wäßrigen Mischung und durch Sprühtrocknung (die Austrittstemperaturen betragen in der Regel 100 bis 150°C, die Eintrittstemperaturen liegen häufig bei 220 bis 340°C; die Sprühtrocknung kann im Gleichstrom oder im Gegenstrom durchgeführt werden), die ein besonders inniges Trockengemisch bedingt, vor allem dann, wenn es sich bei der sprühzutrocknenden wäßrigen Masse um eine wäßrige Lösung handelt.

Als Quellen für die elementaren Konstituenten kommen im Rahmen der Durchführung der vorstehend beschriebenen Herstellweise der katalytisch aktiven Oxidmassen der allgemeinen Formel (I) alle diejenigen in Betracht, die beim Erhitzen (gegebenenfalls an Luft) Oxide und/oder Hydroxide zu bilden vermögen. Selbstredend können als solche Ausgangsverbindungen auch bereits Oxide und/ oder Hydroxide der elementaren Konstituenten mitverwendet oder ausschließlich verwendet werden.

Erfindungsgemäß geeignete Quellen für das Element Mo sind z.B. Molybdänoxide wie Molybdäntrioxid, Molybdate wie Ammoniumheptamolybdattetrahydrat und Molybdänhalogenide wie Molybdänchlorid.

Geeignete, erfindungsgemäß mitzuverwendende Ausgangsverbindungen für das Element V sind z.B. Vanadylacetylacetonat, Vanadate wie Ammoniummetavanadat, Vanadinoxide wie Vanadinpentoxid ($V_2O_5$), Vanadinhalogenide wie Vanadintetrachlorid ($VCl_4$) und Vanadinoxyhalogenide wie $VOCl_3$. Dabei können als Vanadinausgangsverbindungen auch solche mitverwendet werden, die das Vanadin in der Oxidationsstufe +4 enthalten.

Als Quellen für das Element Tellur eignen sich erfindungsgemäß Telluroxide wie Tellurdioxid, metallisches Tellur, Tellurhalogenide wie $TeCl_2$, aber auch Tellursäuren wie Orthothellursäure $H_6TeO_6$.

Erfindungsgemäß geeignete Niobquellen sind z. B. Nioboxide wie Niobpentoxid ($Nb_2O_5$), Nioboxidhalogenide wie $NbOCl_3$, Niobhalogenide wie $NbCl_5$, aber auch komplexe Verbindungen aus Niob und organischen Monocarbonsäuren und/oder Polycarbonsäuren wie z. B. Citrate, Oxalate, aber auch Niobalkoholate- Selbstredend kommen als Niobquelle auch die in der EP-A 895 809 verwendeten Nb enthaltenden Lösungen in Betracht.

Bezüglich aller anderen möglichen Elemente $M^2$ kommen als erfindungsgemäß geeignete Ausgangsverbindungen vor allem deren Halogenide, Nitrate, Formiate, Oxalate, Acetate, Citrate, Carbonate und/oder Hydroxide in Betracht. Geeignete Ausgangsverbindungen sind vielfach auch deren Oxoverbindungen wie z. B. Wolframate bzw. die von diesen abgeleiteten Säuren. Häufig werden als Ausgangsverbindungen auch Ammoniumsalze eingesetzt.

Ferner kommen als Ausgangsverbindungen für die Herstellung der erfindungsgemäßen katalytisch aktiven Oxidmassen der allgemeinen Formel (I) auch Polyanionen vom Anderson Typ in Betracht, wie sie z.B. in JP-A 2000-143244 und in Polyhedron Vol. 6, No. 2, pp. 213-218, 1987 beschrieben sind. Eine weitere geeignete Literaturquelle für Polyanionen vom Anderson Typ bildet Kinetics and Catalysis, Vol. 40, No. 3, 1999, pp 401 bis 404.

Andere als Ausgangsverbindungen geeignete Polyanionen sind z.B. solche vom Dawson oder Keggin Typ. Vorzugsweise werden erfindungsgemäß solche Ausgangsverbindungen verwendet, die sich bei erhöhten Temperaturen entweder im Beisein oder bei Ausschluß von Sauerstoff unter Freisetzung gasförmiger Verbindungen in ihre Oxide umwandeln.

Erfindungsgemäß bevorzugt sind Schalenkatalysatoren, deren katalytisch aktive Oxidmasse der allgemeinen Formel (I) ein Röntgendiffraktogramm aufweist (Cu-$K_\alpha$-Strahlung), das Beugungreflexe h, i und gegebenenfalls k enthält, deren Scheitelpunkte bei den Beugungswinkeln (2Θ) 22,2 ± 0,4° (h), 27,3 ± 0,4° (i) und 28,2 ± 0,4° (k) liegen.

Erfindungsgemäß günstig ist es, wenn dabei der Beugungsreflex h innerhalb des Röntgendiffraktogramms der intensitätsstärkste ist.

Außerdem ist es erfindungsgemäß vorteilhaft, wenn der Beugungsreflex h eine Halbwertsbreite von höchstens 0,5° aufweist.

Die Definition der Intensität eines Beugungsreflexes im Röntgendiffraktogramm bezieht sich in dieser Schrift auf die in der DE-A 19835247 niedergelegte Definition.

D.h., bezeichnet $A^1$ den Scheitelpunkt eines Reflexes 1 und bezeichnet $B^1$ in der Linie des Röntgendiffraktogramms bei Betrachtung entlang der zur 2Θ-Achse senkrecht stehenden Intensitätsache das nächstliegende ausgeprägte Minimum (Reflexschultern ausweisende Minima bleiben unberücksichtig) links vom Scheitelpunkt $A^1$ und $B^2$ in entsprechender Weise das nächstliegende ausgeprägte Minimum rechts vom Scheitelpunkt $A^1$ und bezeichnet $C^1$ den Punkt, an dem eine vom Scheitelpunkt $A^1$ senkrecht zur 2Θ-Achse gezogene Gerade eine die Punkte $B^1$ und $B^2$ verbindende Gerade schneidet, dann ist die Intensität des Reflexes 1 die Länge des Geradenabschnitts $A^1C^1$, der sich vom Scheitelpunkt $A^1$ zum Punkt $C^1$ erstreckt. Der Ausdruck Minimum bedeutet dabei einen Punkt, an dem der Steigungsgradient einer an die Kurve in einem Basisbereich des Reflexes 1 angelegten Tangente von einem negativen Wert auf einen positiven Wert übergeht, oder einen Punkt, an dem der Steigungsgradient gegen Null geht, wobei für die Festlegung des Steigungsgradienten die Koordinaten der 2Θ Achse und der Intensitätsachse herangezogen werden.

Die Halbwertsbreite ist in dieser Schrift in entsprechender Weise die Länge des Geradenabschnitts, der sich zwischen den beiden Schnittpunkten $H^1$ und $H^2$ ergibt, wenn man in der Mitte des Geradenabschnitts $A^1C^1$ eine Parallele zur 2Θ-Achse zieht, wobei $H^1$, $H^2$ den jeweils ersten Schnittpunkt dieser Parallelen mit der wie vorstehend definierten Linie des Röntgendiffraktogramms links und rechts von $A^1$ meinen.

Eine beispielhafte Durchführung der Bestimmung von Halbwertsbreite und Intensität zeigt auch die Figur 6 in der DE-A 10046672.

Darüber hinaus ist es bevorzugt, wenn die Intensität $P_i$ des Beugungsreflexes i und die Intensität $P_k$ des Beugungreflexes k die Beziehung $0,2 \leq R \leq 1$ (bevorzugt $0,5 \leq R \leq 1$ und besonders bevorzugt $0,8 \leq R \leq 1$) erfüllen, in der R das durch die Formel

$$R = P_i / (P_i + P_k)$$

definierte Intensitätsverhältnis ist.

Weiterhin ist es von Vorteil, wenn die Halbwertsbreite des Beugungsreflexes i und eines enthaltenen Beugungsreflexes k jeweils $\leq 1°$ beträgt.

Besonders vorteilhaft ist es, wenn das Röntgendiffraktogramm so beschaffen ist, dass die vorgenannten Randbedingungen simultan erfüllt sind.

Neben den Beugungsreflexen h, i und gegebenenfalls k enthält das Röntgendiffraktogramm vorteilhafter katalytisch aktiver Oxidmassen der allgemeinen Formel (I) noch weitere Beugungsreflexe, deren Scheitelpunkte bei den nachfolgenden Beugungswinkeln (2Θ) liegen:

$$9,0 \pm 0,4° \ (l),$$

$$29,2 \pm 0,4° \ (m)$$

und

$$35,4 \pm 0,4° \ (n).$$

Günstig ist es, wenn das Röntgendiffraktogramm der katalytisch aktiven Oxidmassen der allgemeinen Formel (I) zusätzliche Beugungsreflexe enthält, deren Scheitelpunkte bei folgenden Beugungswinkeln (2Θ) liegen:

$$6,7 \pm 0,4° \ (o),$$

$$7,9 \pm 0,4° \ (p),$$

$$45,2 \pm 0,4° \ (q).$$

Enthält das Röntgendiffraktogramm der katalytisch aktiven Oxidmassen der allgemeinen Formel (I) den Beugungsreflex k, enthält es in der Regel noch weiter Beugungsreflexe, deren Scheitelpunkte bei den nachfolgenden Beugungswinkeln (2θ) liegen:

$$36,2 \pm 0,4°$$

und

$$50,0 \pm 0,4°.$$

Ordnet man dem Beugungsreflex h die Intensität 100 zu, ist es günstig, wenn die Beugungsreflexe i, 1, m, n, o, p, q in der gleichen Intensitätsskala die nachfolgenden Intensitäten aufweisen:

i:      5 bis 95, häufig 5 bis 80, teilweise 10 bis 60;

l:      1 bis 30;

m:     1 bis 40;

n:      1 bis 40;

o:      1 bis 30;

p:      1 bis 30 und

q:      5 bis 60.

Enthält das Röntgendiffraktogramm von den vorgenannten zusätzlichen Beugungsreflexen, ist die Halbertsbreite derselben in der Regel ≤ 1°.

Alle in dieser Schrift auf ein Röntgendiffraktogramm bezogenen Angaben beziehen sich auf ein unter Anwendung von Cu-Kα-Strahlung als Röntgenstrahlung erzeugtes Röntgendiffraktogramm (Siemens-Diffraktometer Theta-Theta D-5000, Röhrenspannung: 40 kV, Röhrenstrom: 40 mA, Aperturblende V20 (variabel), Streustrahlblende V20 (variabel), Sekundärmonochromatorblende (0,1 mm), Detektorblende (0,6 mm), Meßintervall (2θ): 0,02°, Meßzeit je Schritt: 2,4 s, Detektor: Scintillationszählrohr).

Erfindungsgemäß von Bedeutung ist aber auch , dass die Herstellung der erfindungsgemäßen Schalenkatalysatoren nicht nur durch Aufbringen der fertiggestellten, feingemahlenen aktiven Oxidmassen der allgemeinen Formel (I) auf die befeuchtete Trägerkörperoberfläche erfolgen kann.

Vielmehr kann anstelle der aktiven Oxidmasse auch eine feinteilige Vorläufermasse derselben auf die befeuchtete Trägeroberfläche (unter Anwendung der gleichen Beschichtungsverfahren und Bindemittel) aufgebracht und die Calcination nach Trocknung des beschichteten Trägerkörpers durchgeführt werden.

Als eine solche feinteilige Vorläufermasse kommt z. B. diejenige Masse in Betracht, die dadurch erhältlich ist, dass man aus den Quellen der elementaren Konstituenten der gewünschten aktiven Oxidmasse der allgemeinen Formel (I) zunächst ein möglichst inniges, vorzugsweise feinteiliges, Trockengemisch erzeugt (z. B. durch Sprühtrocknung einer wäßrigen Suspension oder Lösung der Quellen) und dieses feinteilige Trockengemisch (gegebenenfalls nach Tablettierung unter Zusatz von 0,5 bis 2 Gew.-% an feinteiligem Graphit) bei einer Temperatur von 150 bis 350°C, vorzugsweise 250 bis 350°C unter oxidierender (Sauerstoff enthaltender) Atmosphäre (z. B. unter Luft) thermisch behandelt (wenige Stunden) und abschließend bei Bedarf einer Mahlung unterwirft.

Nach der Beschichtung der Trägerkörper mit der Vorläufermasse wird dann, bevorzugt unter Inertgasatmosphäre (alle anderen Atmosphären kommen auch in Betracht) bei Temperaturen von 360 bis 700°C bzw. 400 bis 650°C oder 400 bis 600°C calciniert.

Im übrigen kann die Anwendung der erfindungsgemäßen Schalenkatalysatoren als Katalysatoren für die gasphasenkatalytische Oxidation von Propan zu Acrylsäure im wesentlichen wie die in der EP-A 962253, EP-A 608838, der WO 00/29106 sowie der JP-A 10-36311 beschriebenen Verfahrensweisen durchgeführt werden.

D.h., das Reaktionsgasgemisch, mit dem die Schüttung der erfindungsgemäßen Schalenkatalysatoren bei Reaktionstemperaturen von z. B. 200 bis 550°C oder von 230 bis 480°C bzw. 300 bis 440 °C zu belasten ist, kann z. B. nachfolgende Zusammensetzung aufweisen:

1 bis 15, vorzugsweise 1 bis 7 Vol.-% Propan,

44 bis 99 Vol.-% Luft und

0 bis 55 Vol.-% Wasserdampf.

Unter dem Aspekt einer möglichst hohen Selektivität der Acrylsäurebildung sind Wasserdampf enthaltende Reaktionsgasausgangsgemische bevorzugt.

Als andere mögliche Zusammensetzungen des Reaktionsgasausgangsgemisches kommen in Betracht:

70 bis 95 Vol.-% Propan,

5 bis 30 Vol.-% molekularer Sauerstoff und

0 bis 25 Vol.-% Wasserdampf.

Die Durchführung der heterogen katalysierten Gasphasenoxidation von Propan zu Acrylsäure unter Verwendung der erfindungsgemäßen Schalenkatalysatoren kann in an sich bekannter Weise in mit Salzbad gekühlten Rohrbündelreaktoren durchgeführt werden, wie sie für die heterogen katalysierte Gasphasenoxidation von Propen zu Acrolein bzw. von Acrolein zu Acrylsäure bekannt und z.B. in der EP-A 700714 sowie in der EP-A 700893 sowie im in den beiden vorgenannten Schriften zitierten Stand der Technik beschrieben sind. Reaktionsgasgemisch und Salzbad können dabei sowohl im Gleichstrom als auch im Gegenstrom durch den Reaktor geführt werden. Dem Salzbad kann dabei zusätzlich eine Querströmung überlagert werden. Bei Bedarf kann man das Salzbad mäanderförmig um die Kontaktrohre führen.

Selbstredend wird beim erfindungsgemäßen Verfahren ein Produktgasgemisch erhalten, das nicht ausschließlich aus Acrylsäure besteht. Vielmehr enthält das Produktgasgemisch neben nicht umgesetztem Propan Nebenkomponenten wie Propen, Acrolein, $CO_2$, CO, $H_2O$, Essigsäure, Propionsäure etc., von denen die Acrylsäure abgetrennt werden muß.

Dies kann so erfolgen, wie es von der heterogen katalysierten Gasphasenoxidation von Propen zu Acrylsäure bekannt ist.

D.h., aus dem Produktgasgemisch kann die enthaltene Acrylsäure durch Absorption mit Wasser oder durch die Absorption mit einem hochsiedenden inerten hydrophoben organischen Lösungsmittel (z.B. einem Gemisch aus Diphenylether und Diphyl das gegebenenfalls noch Zusätze wie Dimethylphthalat enthalten kann) aufgenommen werden. Das dabei resultierende Gemisch aus Absorbens und Acrylsäure kann anschließend in an sich bekannter Weise rektifikativ, extraktiv und/oder kristallisativ bis zur Reinacrylsäure aufgearbeitet werden. Alternativ kann die Grundabtrennung der Acrylsäure aus dem Produktgasgemisch auch durch fraktionierte Kondensation erfolgen, wie es z.B. in der DE-A 19 924 532 beschrieben ist.

Das dabei resultierende wäßrige Acrylsäurekondensat kann dann z.B. durch fraktionierte Kristallisation (z.B. Suspensionskristallisation und/oder Schichtkristallisation) weitergereinigt werden.

Das bei der Grundabtrennung der Acrylsäure verbleibende Restgasgemisch enthält insbesondere nicht umgesetztes Propan. Dies kann aus dem Restgasgemisch z.B. durch fraktionierte Druckrektifikation abgetrennt und anschließend in die erfindungsgemäße Gasphasenoxidation rückgeführt werden. Günstiger ist es jedoch, das Restgas in einer Extraktionsvorrichtung mit einem hydrophoben organischen Lösungsmittel in Kontakt zu bringen (z.B. durch selbiges durchleiten), das das Propan bevorzugt zu absorbieren vermag.

Durch nachfolgende Desorption und/oder Strippung mit Luft kann das absorbierte Propan wieder freigesetzt und in das erfindungsgemäße Verfahren rückgeführt werden. Auf diese Weise sind wirtschaftliche Gesamtpropanumsätze erzielbar.

Erfindungsgemäß wesentlich ist, dass die erfindungsgemäßen Schalenkatalysatoren eine höhere Selektivität der Acrylsäurebildung bedingen als Splitt aus aktiven Oxidmassen der allgemeinen Formel I.

Die erfindungsgemäßen Schalenkatalysatoren eignen sich auch zur gasphasenkatalytisch oxidativen Herstellung von Methacrylsäure aus deren $C_4$-Vorläufern wie z.B. n-Butan, iso-Butan oder iso-Buten sowie zur gasphasenkatalytisch oxidativen Herstellung von Acrylsäure aus Propen. Abschließend sei festgehalten, dass die im Rahmen einer gasphasenkatalytisch oxidativen Propanoxidation zu Acrylsäure bzw. Butanoxidation zu Methacrylsäure verbrauchten erfindungsgemäßen Schalenkatalysatoren wie in der EP-A 339119 beschrieben regeneriert werden können.

Vergleichsbeispiel und Beispiel

Vergleichsbeispiel:

1287,25 g Ammoniummetavanadat (77,5 Gew.-% $V_2O_5$, Fa. G.f.E. Nürnberg, DE) wurden bei 80°C in einem Edelstahlbehälter unter Rühren gelöst. Es entstand eine gelbliche klare Lösung. Diese Lösung wurde auf 60°C abgekühlt und dann unter Aufrechterhaltung der 60°C in der genannten Reihenfolge nacheinander 1683,75 g Tellursäure (99 Gew.-% $H_6TeO_6$, Fa. Fluka, DE) und 5868,0 g Ammoniumheptamolybdat (81,5 Gew.-% $MoO_3$, Fa. Starck, DE) in die Lösung eingerührt. Es entstand eine tiefrote Lösung A.

In einem zweiten beheizbaren Topf aus Edelstahl wurden unter Rühren bei 60°C 1599 g Ammoniumnioboxalat (21,1 Gew.-% Nb, Fa. Starck, DE) in 8,3 l Wasser gelöst (Lösung B).

Die Lösung A und die Lösung B wurden auf 30°C abgekühlt und bei dieser Temperatur unter Rühren vereinigt, wobei die Lösung B zur Lösung A gegeben wurde. Die Zugabe erfolgte stetig über einen Zeitraum von 10 min. Es entstand eine orangefarbene Suspension.

Diese Suspension wurde anschließend in einem Sprühtrockner der Firma Niro (Sprühtrockner Niro A/S Atomizer, Transprotable Minor Anlage, Zentrifugalzerstäuber der Fa. Niro, DK) sprühgetrocknet. Die Vorlagetemperatur betrug 30°C. Die Gaseintrittstemperatur $T^{ein}$ betrug 240°C, die Gasaustrittstemperatur $T^{aus}$ betrug 110°C. Das resultierende Sprühpulver war ebenfalls orangefarben.

Unter Zusatz von 1 Gew.-% Graphit wurde das Sprühpulver zu Ringtabletten der Außenmaße 16 mm x 2,5 mm x 8 mm (Außendurchmesser x Höhe x Innendurchmesser) verpresst (der angewendete Pressdruck betrug 50 MPa, die resultierende Seitendruckfestigkeit lag bei 10 N).

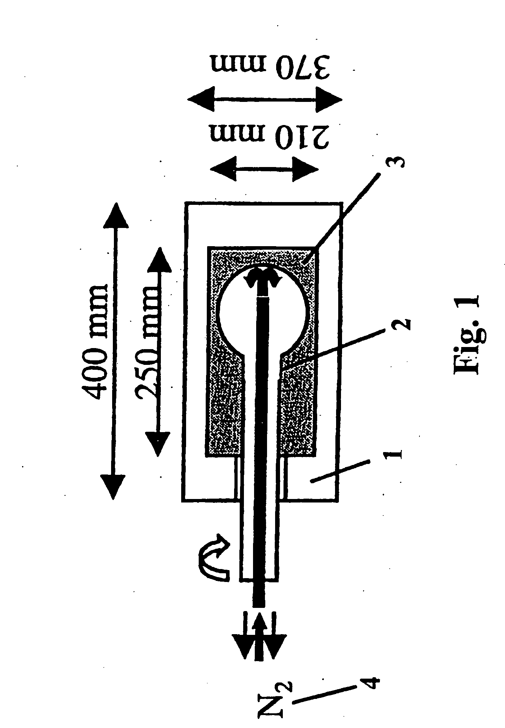

100 g dieser Ringe wurden in einem Drehkugelofen gemäß beiliegender Figur 1 (1 = Ofengehäuse, 2 = Drehkolben, 3 = beheizter Raum, 4 = Stickstoff-/Luftstrom) kalziniert, indem zunächst innerhalb von 27,5 min unter einem Luftstrom von 50 Nl/h von 25°C linear auf 275°C erhitzt wurde. Diese Temperatur wurde anschließend unter Beibehalt des Luftstroms während 1 h aufrechterhalten.

Dann wurde der Luftstrom durch einen Stickstoffstrom von 50 Nl/h ersetzt und die Calcinationstemperatur innerhalb von 32,5 min linear von 275°C auf 600°C erhöht. Diese Temperatur wurde dann für 2 h aufrechterhalten und abschließend der gesamte Drehkugelofen durch sich selbst überlassen auf Raumtemperatur abgekühlt.

Es resultierten schwarze Tabletten der Zusammensetzung $Mo_{1,0} V_{0,33} Te_{0,15} Nb_{0,11} O_x$ (Einwaagestöchiometrie: $Mo_{1,0} V_{0,33} Te_{0,22} Nb_{0,11} O_x$).

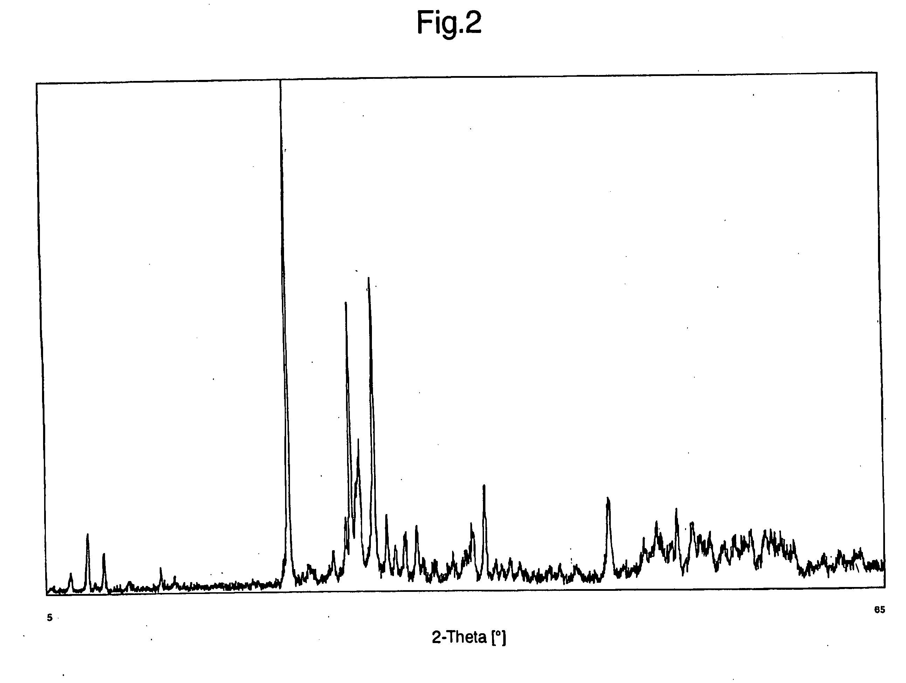

Das zugehörige Röntgendiffraktogramm zeigt die beiliegende Figur 2.

Die erhaltenen Tabletten aus oxidischer Aktivmasse wurden im Mörser zu Splitt verstoßen und die Konfraktion, die durch quadratische Siebmaschen mit einer Kantenlänge von 1,2 mm noch durchfällt, von quadratischen Siebmaschen mit einer Kartenlänge von 0,6 mm aber nicht mehr durchgelassen wird, durch Sieben abgetrennt.

Mit 7 g der abgetrennten Siebfraktion wurde ein aus Stahl gefertigter Rohrreaktor (Innendurchmesser: 8,5 mm, Länge: 140 cm, Wanddicke: 2,5 cm) beschickt (Katalysatorschüttlange = 11 cm). Vor der Schüttung aus aktiver Oxidmasse wurde eine Vorschüttung von 30 cm Quarzsplitt (Körnung: 1 - 2 mm) und nach der Schüttung aus aktiver Oxidmasse auf der Restlänge des Rohrreaktors eine Nachschüttung desselben Quarzsplitt angebracht.

Mittels elektrisch beheizter Heizmatten wurde von außen die Außenwandtemperatur des beschickten Reaktionsrohres auf der gesamten Länge auf 350°C eingestellt. Dann wurde das Reaktionsrohr mit einem Reaktionsgasausgangsgemisch der molaren Zusammensetzung Propan: Luft: $H_2O$ = 1:15:14 beschickt (die Eintrittsseite war auf der Seite der Nachschüttung). Die Verweilzeit (bezogen auf die Aktivmassenschüttung) wurde auf 2,4 sec eingestellt. Der Eingangsdruck betrug 2 bar absolut.

Nach einer Betriebsdauer von 42 h lag der Propanumsatz bei einfachem Durchgang bei 24 mol-%. Die Selektivität der Acrylsäurebildung betrug 53 mol-%. Zusätzlich wurde mit einer Selektivität von 10 mol-% Propen als Wertnebenprodukt gebildet.

Beispiel:

Es wurde zwei Mal wie im Vergleichsbeispiel vorgegangen und so die doppelte Menge schwarzer Ringtabletten aus oxidischer Aktivmasse hergestellt.

Die Ringtabletten aus oxidischer Aktivmasse wurden in einer Retsch-Mühle trocken zu einem Pulver aufgemahlen, von dem 50 % der Pulverpartikel ein Sieb der Maschenweite 1 bis 20 $\mu$m passierten und dessen numerischer Anteil an Partikeln mit einer Längstausdehnung oberhalb von 50 $\mu$m weniger als 10 % betrug. 0,6 kg Steatitkugeln (Steatit-Splittgranulat, Durchmesser 2,5 - 3,2 mm, Art.Nr. 1.080023.60.00.00, Oberflächenrauhigkeit $R_z$ = 45 $\mu$m, auf das Volumen der Trägerkörper bezogenes Porengesamtvolumen $\leq$ 1 Vol.-%, Hersteller: Hoechst Ceramtec, Steatit-splittgranulat, Durchmesser 2,5 - 3,2 mm, Art.Nr. 1.080023.60.00.00 DE) wurden in einem Dragierkessel (Neigungswinkel: 45°; Hicoater der Fa. Lödige, DE) von 3 1 Innenvolumen gefüllt.

Anschließend wurde der Dragierkessel mit 16 U/min in Rotation versetzt. Über eine Düse wurden innerhalb von 30 min 50 ml eines Gemisches aus Glycerin und Wasser (Gewichtsverhältnis Glycerin: Wasser = 1:3) auf die kugelförmigen Trägerkörper aufgesprüht. Gleichzeitig wurden im selben Zeitraum 156 g des aufgemahlenen Ringtablettenpulvers über eine Schüttelringe außerhalb des Sprühkegels der Zerstäuberdüse kontinuierlich zudosiert. Während der Beschichtung wurde das zugeführte Pulver vollständig auf die Oberfläche der Trägerkörper aufgenommen. Eine Agglomeration der feinteiligen oxidischen Aktivmasse wurde nicht beobachtet. Die beschichteten Trägerkörper wurden noch 16 h bei 150°C im Muffelofen getrocknet.

Die resultierenden Schalenkatalysatoren wiesen eine Aktivmassenschichtdicke von 180 $\mu$m auf. Dies entsprach einem Aktivmassenanteil der Schalenkatalysatoren von 20 Gew.-%.

Mit 35,0 g des Schalenkatalysators wurde ein aus Stahl gefertigter Rohrreaktor (Innendurchmesser: 8,5 mm, Länge 1,40 cm, Wanddicke: 2,5 cm) beschickt (Katalysatorschüttlänge = 55 cm). Vor der Katalysatorschüttung wurde eine Vorschüttung von 30 cm Quarzsplitt (Körnung: 1 - 2 mm) und nach der Katalysatorschüttung auf der Restlänge des Rohrreaktors eine Nachschüttung desselben Quarzsplitt angebracht.

Mittels elektrisch beheizter Heizmatten wurde von außen die Außentemperatur des beschickten Reaktionsrohres auf der gesamten Länge auf 350 °C eingestellt.

Dann wurde das Reaktionsrohr mit einem Reaktionsgasausgangsgemisch der molaren Zusammensetzung Propan: Luft: $H_2O$ = 1:15:14 beschickt (die Eintrittsseite war auf der Seite der Nachschüttung). Die Verweilzeit (bezogen auf die Aktivmassenschüttung) wurde auf 2,4 sec eingestellt. Der Eingangdruck betrug 2 bar absolut.

Nach einer Betriebsdauer von 42 h lag der Propanumsatz bei einfachem Durchgang bei 25 mol-%. Die Selektivität der Acrylsäurebildung betrug 58 mol-%. Zusätzlich wurde mit einer Selektivität von 13 mol-% Propen als Wertnebenprodukt gebildet.

**Patentansprüche**

1. Schalenkatalysator, der aus einem Trägerkörper und einer auf der Oberfläche des Trägerkörpers aufgebrachten katalytisch aktiven Oxidmasse der allgemeinen Formel I,

$$Mo_1V_bM^1_cM^2_dO_n \qquad (I),$$

mit

M$^1$ = Te
M$^2$ = wenigstens eines der Elemente aus der Gruppe umfassend Nb, Ta, W, Ti, Al, Zr, Cr, Mn, Ga, Fe, Ru, Co, Rh, Ni, Pd, Pt, Bi, B und Ce,
b = 0,01 bis 1,
c = 0,01 bis 1,
d = 0,01 bis 1 und
n = eine Zahl, die unter der Voraussetzung der Ladungsneutralität durch die Wertigkeit und Häufigkeit der von Sauerstoff verschiedenen Elemente in (I) bestimmt wird,

besteht, **dadurch gekennzeichnet, dass** die Längstausdehnung des Trägerkörpers 1 bis 10 mm und die Dicke der aktiven Oxidmassenschale 10 bis 700 $\mu$m beträgt.

2. Schalenkatalysator nach Anspruch 1, wobei M$^2$ = Nb.

3. Schalenkatalysator nach Anspruch 1 oder 2 mit b = 0,1 bis 0,6.

4. Schalenkatalysator nach einem der Ansprüche 1 bis 3 mit c = 0,05 bis 0,4.

5. Schalenkatalysator nach einem der Ansprüche 1 bis 4 mit d = 0,1 bis 0,6.

6. Schalenkatalysator nach einem der Ansprüche 1 bis 5, wobei der Trägerkörper aus Aluminiumoxid, Siliciumdioxid, Ton, Kaolin, Steatit, Bims, Aluminiumsilicat, Magnesiumsilicat, Siliciumcarbid, Zirkondioxid oder Thoriumdioxid besteht.

7. Schalenkatalysator nach einem der Ansprüche 1 bis 6, wobei der Trägerkörper Kugel- oder Zylindergeometrie aufweist.

8. Schalenkatalysator nach Anspruch 7, wobei der Trägerkörper eine Kugel mit einem Durchmesser von 1 bis 10 mm ist.

9. Schalenkatalysator nach Anspruch 7, wobei der Trägerkörper ein Ring mit einer Länge von 2 bis 10 mm, einem Außendurchmesser von 4 bis 10 mm und einer Wanddicke von 1 bis 4 mm ist.

10. Schalenkatalysator nach einem der Ansprüche 1 bis 9, wobei die katalytisch aktive Oxidmasse ein Röntgendiffraktogramm aufweist, das Beugungsreflexe h, i und gegebenenfalls k enthält, deren Scheitelpunkte bei den Beugungswinkeln (2θ) 22,2 ± 0,4° (h), 27,3 ± 0,4° (i) und 28,2 ± 0,4° (k) liegen.

11. Schalenkatalysator nach Anspruch 10, wobei der Beugungsreflex h innerhalb des Röntgendiffraktogramms der intensitätsstärkste ist.

12. Schalenkatalysator nach Anspruch 10, wobei der Beugungsreflex h eine Halbwertsbreite von höchstens 0,5° aufweist.

13. Schalenkatalysator nach einem der Ansprüche 10 bis 12, wobei die Intensität $P_i$ des Beugungsreflexes i und die Intensität $P_k$ des Beugungsreflexes k die Beziehung $0,2 \leq R \leq 1$ erfüllen, in der R das durch die Formel

$$R = P_i / (P_i + P_k)$$

definierte Intensitätsverhältnis ist.

14. Schalenkatalysator nach einem der Ansprüche 1 bis 13, wobei die katalytisch aktive Oxidmasse in einer Schichtdicke von 100 bis 700 $\mu$m auf die Oberfläche des Trägerkörpers aufgebracht ist.

15. Verfahren der gasphasenkatalytischen Oxidation von Propan zu Acrylsäure, wobei als Katalysator ein solcher gemäß einem der Ansprüche 1 bis 14 verwendet wird.

16. Verwendung eines Schalenkatalysators gemäß einem der Ansprüche 1 bis 14 zur gasphasenkatalytischen Oxidation von Propan zu Acrylsäure.

17. Verfahren zur Herstellung eines Schalenkatalysators, gemäß einem der Ansprüche 1 bis 14, das **dadurch gekennzeichnet ist, dass** man eine katalytisch aktive Oxidmasse der allgemeine Formel (I) in feinteiliger Form vorbildet, den Trägerkörper mit einem flüssigen Bindemittel befeuchtet, danach durch Inkontaktbringen mit feinteiliger katalytisch aktiver Oxidmasse der allgemeinen Formel (I) an der Oberfläche des befeuchteten Trägerkörpers eine Schicht aktiver Oxidmasse anheftet und anschließend den beschichteten Trägerkörper trocknet.

18. Verfahren zur Herstellung eines Schalenkatalysators, gemäß Anspruch 17, das **dadurch gekennzeichnet ist, dass** man aus Ausgangsverbindungen der elementaren Konstituenten der katalytisch aktiven Oxidmasse ein inniges Trockengemisch erzeugt, das innige Trockengemisch bei einer Temperatur von 150 bis 350 °C unter Erhalt einer Vorläufermasse unter oxidierender Atmosphäre thermisch behandelt, den Trägerkörper mit flüssigem Bindemittel befeuchtet, danach durch Inkontaktbringen mit feinteiliger Vorläufermasse an der Oberfläche des befeuchteten Trägerkörpers eine Schicht der Vorläufermasse anheftet, anschließend den beschichteten Trägerkörper trocknet und abschließend den getrockneten, mit Vorläufermasse beschichteten. Trägerkörper bei einer Temperatur von 400 bis 650 °C calciniert.

**Claims**

1. A coated catalyst comprising a support body and a catalytically active oxide composition of the formula I

$$Mo_1V_bM_cM_dO_n \qquad (I)$$

where

$M^1$ = Te
$M^2$ = at least one element selected from the group consisting of Nb, Ta, W, Ti, Al, Zr, Cr, Mn, Ga, Fe, Ru, Co, Rh, Ni, Pd, Pt, Bi, B and Ce,
b = 0.01 to 1,
c = 0.01 to 1,
d = 0.01 to 1 and
n = a number required to achieve electrical neutrality and determined by the valence and abundance of the elements other than oxygen in (I),

applied to the surface of the support body, wherein the longitudinal dimension of the support body is from 1 to 10 mm and the thickness of the active oxide composition layer is from 10 to 700 $\mu$m.

2. A coated catalyst according to claim 1, wherein $M^2$ = Nb.

3. A coated catalyst according to claim 1 or 2, wherein b = 0.1 to 0.6.

4. A coated catalyst according to any of claims 1 to 3, wherein c = 0.05 to 0.4.

5. A coated catalyst according to any of claims 1 to 4, wherein d = 0.1 to 0.6.

6. A coated catalyst according to any of claims 1 to 5, wherein the support body comprises aluminum oxide, silicon dioxide, clay, kaolin, steatite, pumice, aluminum silicate, magnesium silicate, silicon carbide, zirconium dioxide or thorium dioxide.

7. A coated catalyst according to any of claims 1 to 6, wherein the support body has a spherical or cylindrical geometry.

8. A coated catalyst according to claim 7, wherein the support body is a sphere having a diameter of from 1 to 10 mm.

9. A coated catalyst according to claim 7, wherein the support body is a ring having a length of from 2 to 10 mm, an external diameter of from 4 to 10 mm and a wall thickness of from 1 to 4 mm.

10. A coated catalyst according to any of claims 1 to 9, wherein the catalytically active oxide composition has an X-ray diffraction pattern which displays reflections h, i and optionally k whose maxima are at 2$\Theta$ values of 22.2 $\pm$ 0.4° (h), 27.3 $\pm$ 0.4° (i) and 28.2 $\pm$ 0.4° (k).

11. A coated catalyst according to claim 10, wherein the reflection h is the most intense reflection in the X-ray diffraction pattern.

12. A coated catalyst according to claim 10, wherein the reflection h has a width at half height of not more than 0.5°.

13. A coated catalyst according to any of claims 10 to 12, wherein the intensity $P_i$ of the reflection i and the intensity $P_k$ of the reflection k obey the relationship 0.2 $\leq$ R $\leq$ 1, where R is the intensity ratio defined by the formula

$$R = P_i / (P_i + P_k).$$

**14.** A coated catalyst according to any of claims 1 to 13, wherein the catalytically active oxide composition is applied in a layer thickness of from 100 to 700 μm to the surface of the support body.

**15.** A process for the gas-phase catalytic oxidation of propane to acrylic acid, wherein the catalyst used is a catalyst according to any of claims 1 to 14.

**16.** The use of a coated catalyst according to any of claims 1 to 14 for the gas-phase catalytic oxidation of propane to acrylic acid.

**17.** A process for producing a coated catalyst according to any of claims 1 to 14, which comprises preforming a catalytically active oxide composition of the formula (I) in finely divided form, moistening the support body with a liquid binder, then applying a layer of active oxide composition to the surface of the moistened support body by bringing it into contact with finely divided catalytically active oxide composition and subsequently drying the coated support body.

**18.** A process for producing a coated catalyst according to claim 17, which comprises producing an intimate dry mixture from starting compounds of the elemental constituents of the catalytically active oxide composition, treating the intimate dry mixture thermally at from 150 to 350°C under an oxidizing atmosphere to give a precursor composition, moistening the support body with a liquid binder, then applying a layer of the precursor composition to the surface of the moistened support body by bringing it into contact with finely divided precursor composition, subsequently drying the coated support body and finally calcining the support body coated with precursor composition at from 400 to 650°C.

**Revendications**

**1.** Catalyseur à enveloppe constitué par un corps support et une masse d'oxyde catalytiquement active appliquée sur la surface du corps support, de formule générale I

$$Mo_1V_bM^1{}_cM^2{}_dO_n \qquad (I)$$

avec

M$^1$ = Te,
M$^2$ = au moins un des éléments du groupe comprenant Nb, Ta, W, Ti, Al, Zr, Cr, Mn, Ga, Fe, Ru, Co, Rh, Ni, Pd, Pt, Bi, B et Ce,
b = 0,01 à 1,
c = 0,01 à 1,
d = 0,01 à 1 et
n = un nombre déterminé par la valence et la fréquence des éléments différents de l'oxygène dans (I) en supposant la neutralité des charges,

**caractérisé en ce que** la dimension longitudinale du corps support est de 1 à 10 mm et l'épaisseur de l'enveloppe de masse d'oxyde active est de 10 à 700 μm.

**2.** Catalyseur à enveloppe selon la revendication 1, dans lequel M$^2$ = Nb.

**3.** Catalyseur à enveloppe selon la revendication 1 ou 2, avec b = 0,1 à 0,6.

**4.** Catalyseur à enveloppe selon l'une quelconque des revendications 1 à 3, avec c = 0,05 à 0,4.

**5.** Catalyseur à enveloppe selon l'une quelconque des revendications 1 à 4, avec d = 0,1 à 0,6.

**6.** Catalyseur à enveloppe selon l'une quelconque des revendications 1 à 5, dans lequel le corps support est constitué d'oxyde d'aluminium, de dioxyde de silicium, d'argile, de kaolin, de stéatite, de pierre ponce, de silicate d'aluminium, de silicate de magnésium, de carbure de silicium, de dioxyde de zirconium ou de dioxyde de thorium.

7.  Catalyseur à enveloppe selon l'une quelconque des revendications 1 à 6, dans lequel le corps support présente une géométrie sphérique ou cylindrique.

8.  Catalyseur à enveloppe selon la revendication 7, dans lequel le corps support est une sphère d'un diamètre de 1 à 10 mm.

9.  Catalyseur à enveloppe selon la revendication 7, dans lequel le corps support est un anneau d'une longueur de 2 à 10 mm, d'un diamètre extérieur de 4 à 10 mm et d'une épaisseur de paroi de 1 à 4 mm.

10. Catalyseur à enveloppe selon l'une quelconque des revendications 1 à 9, dans lequel la masse d'oxyde catalytiquement active présente un diffractogramme de rayons X qui contient des reflets de diffraction h, i et éventuellement k, dont les pics se situent aux angles de diffraction (2θ) 22,2 ± 0,4° (h), 27,3 ± 0,4° (i) et 28, 2 ± 0,4° (k).

11. Catalyseur à enveloppe selon la revendication 10, dans lequel le reflet de diffraction h dans le diffractogramme de rayons X est le plus intense.

12. Catalyseur à enveloppe selon la revendication 10, dans lequel le reflet de diffraction h présente une largeur à mi-hauteur d'au plus 0,5°.

13. Catalyseur à enveloppe selon l'une quelconque des revendications 10 à 12, dans lequel l'intensité $P_i$ du reflet de diffraction i et l'intensité $P_k$ du reflet de diffraction k satisfont la relation $0,2 \leq R \leq 1$, R étant le rapport d'intensité défini par la formule

$$R = P_i / (P_i + P_k).$$

14. Catalyseur à enveloppe selon l'une quelconque des revendications 1 à 13, dans lequel la masse d'oxyde catalytiquement active est appliquée sur la surface du corps support en une épaisseur de couche de 100 à 700 μm.

15. Procédé d'oxydation catalytique en phase gazeuse de propane en acide acrylique, dans lequel un catalyseur selon l'une quelconque des revendications 1 à 14 est utilisé en tant que catalyseur.

16. Utilisation d'un catalyseur à enveloppe selon l'une quelconque des revendications 1 à 14 pour l'oxydation catalytique en phase gazeuse de propane en acide acrylique.

17. Procédé de fabrication d'un catalyseur à enveloppe selon l'une quelconque des revendications 1 à 14, **caractérisé en ce qu'**une masse d'oxyde catalytiquement active de formule générale (I) est préformée sous forme finement divisée, le corps support est humidifié avec un liant liquide, puis une couche de masse d'oxyde active est fixée sur la surface du corps support humidifié par mise en contact avec la masse d'oxyde catalytiquement active finement divisée de formule générale (I), puis le corps support revêtu est séché.

18. Procédé de fabrication d'un catalyseur à enveloppe selon la revendication 17, **caractérisé en ce qu'**un mélange intime sec est formé à partir de composés de départ des constituants élémentaires de la masse d'oxyde catalytiquement active, le mélange intime sec est traité thermiquement à une température de 150 à 350 °C dans une atmosphère oxydante pour obtenir une masse de précurseur, le corps support est humidifié avec un liant liquide, puis une couche de la masse de précurseur est fixée sur la surface du corps support humidifié par mise en contact avec la masse de précurseur finement divisée, puis le corps support revêtu est séché, et enfin le corps support revêtu avec la masse de précurseur et séché est calciné à une température de 400 à 650 °C.

370 mm

210 mm

400 mm

250 mm

3

2

1

N₂

4

Fig. 1

15

# Fig.2

2-Theta [°]

EP 1 335 793 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 608838 A
- EP 529853 A
- JP 7232071 A
- JP 10057813 A
- JP 2000037623 A
- JP 10036311 A
- EP 767164 A
- EP 962253 A
- JP 11169716 A
- EP 895809 A
- DE 19835247 A
- DE 10029338 A

- DE 10046672 A
- WO 0029106 A
- DE 4442346 A
- EP 1090684 A
- DE 2909671 A
- DE 10033121 A
- JP 2000143244 A
- EP 700714 A
- EP 700893 A
- DE 19924532 A
- EP 339119 A

**In der Beschreibung aufgeführte Nicht-Patentliteratur**

- Procedings ISO'99, Rimini (Italy). SCI Pub, 10. September 1999
- *Catalysis Today,* 1999, vol. 49, 141-153
- *Applied Catalysis A: General,* 2000, vol. 194-195, 479-485

- *Polyhedron,* 1987, vol. 6 (2), 213-218
- *Polyanionen vom Anderson Typ bildet Kinetics and Catalysis,* 1999, vol. 40 (3), 401-404